Europäisches Patentamt

European Patent Office

Office européen des brevets

(19)

(11) Numéro de publication: **0 354 129**
**A1**

(12) **DEMANDE DE BREVET EUROPEEN**

(21) Numéro de dépôt: 89402216.9

(22) Date de dépôt: 04.08.89

(51) Int. Cl.⁵: **C 12 N 1/38**
C 12 N 5/00

(30) Priorité: 05.08.88 FR 8810652

(43) Date de publication de la demande:
07.02.90 Bulletin 90/06

(84) Etats contractants désignés:
AT BE CH DE ES FR GB GR IT LI LU NL SE

(71) Demandeur: SANOFI
40, Avenue George V
F-75008 Paris (FR)

(72) Inventeur: Roasio, Annette
Chemin du Grangé Le Fauga
F-31410 Noe (FR)

Labit-Le Bouteiller, Christine
23, rue de l'Amandier
F-31100 Toulouse (FR)

Lupker, Johannes Hermanus
4 bis, rue Lamartine
F-31320 Castanet Tolosan (FR)

(74) Mandataire: Gillard, Marie-Louise et al
Cabinet Beau de Loménie 55, Rue d'Amsterdam
F-75008 Paris (FR)

(54) Agent activateur de la productivité spécifique des cellules animales à base de polyvinylpyrrolidone et milieu de culture défini le contenant.

(57) La présente invention concerne un agent activateur de productivité spécifique de cellules animales, notamment de cellules recombinantes et les milieux de culture définis le contenant. Cet agent est constitué d'une polyvinylpyrrolidone ayant un poids moléculaire moyen d'au moins 2500 et contient éventuellement en outre un copolymère d'oxyde d'éthylène et d'oxyde de propylène de poids moléculaire moyen compris entre 1100 et 8500 environ.

Application : activation de la production spécifique de cellules animales, par exemple de cellules CHO productrices d'IL-2.

## Description

**Agent activateur de la productivité spécifique des cellules animales à base de polyvinylpyrrolidone et milieu de culture défini le contenant.**

La présente invention se rapporte au domaine de la production de métabolites, en particulier de protéines par voie génétique et concerne plus particulièrement un agent activateur de la productivité spécifique des cellules animales à base de polyvinylpyrrolidone. Elle concerne également les milieux de culture définis contenant l'agent activateur de la productivité spécifique pour la culture de cellules animales, en particulier les cellules animales recombinantes, par exemple les cellules transfectées par un vecteur d'expression codant pour une protéine d'intérêt, et les hybridomes, cellules fusionnées en général dans le but de l'excrétion d'une métabolite d'intérêt, le plus souvent une protéine.

Les milieux de culture sont généralement constitués d'un milieu de base, qui contient les éléments nutritifs essentiels au développement des cellules, complémenté par du sérum sanguin, le plus souvent du sérum de veau foetal.

Les cultures cellulaires sont réalisées soit en suspension, c'est le cas par exemple des hybridomes, soit sur supports, par exemple celles des cellules animales adhérentes.

L'utilisation du sérum sanguin dans les milieux de culture conduit à des milieux de culture non définis dans la mesure où la composition du sérum sanguin varie d'un lot à l'autre et dans laquelle peuvent figurer des composants favorisant ou inhibant la croissance et la production protéique des cellules.

Des recherches ont été entreprises depuis longtemps pour d'une part remplacer le sérum sanguin et d'autre part améliorer les conditions de culture de cellules animales.

Parmi les diverses substances qui ont été proposées à cet effet, on peut citer les biopolymères et les polymères de synthèse, lesquels ont été testés en culture classique comme en fermentation industrielle:
- soit en remplacement du sérum de veau foetal ; ils sont alors utilisés comme agents viscosifiants pouvant jouer sur la pression oncotique des milieux de culture;
- soit comme agents protecteurs ; en effet ils peuvent adsorber différents molécules toxiques ou non. Ce rôle peut être couplé avec un rôle de transporteur puisque certains ont la propriété de pénétrer dans le cellule sans être dégradés ;
- soit pour améliorer le transfert en oxygène dans les fermenteurs.

Les polymères les plus couramment utilisés dans ce domaine sont d'une part l'hydroxyéthylamidon, la carboxyméthylcellulose, la gélatine, les cyclodextrines, le dextrane et d'autre part les polyéthylèneglycols, la polyvinylpyrrolidone et les copolymères d'oxyde de propylène et d'oxyde d'éthylène (connus sous la dénomination commerciale PLURIOL).

A cet effet on peut citer notamment :
- l'article de MIZRAHI dans "Develop. Biol. Standard. Vol. 55 pp. 93-102" qui décrit l'utilisation de polymères tels que la carboxyméthylcellulose, l'hydroxyéthylamidon et les polyols pluroniques comme adjuvants de milieux de culture pour le développement des cellules en suspension. Ces polymères protègent les cellules des dommages mécaniques occasionnés par l'agitation et l'aération des milieux.
- la demande internationale WO 88/00967 qui décrit un milieu de culture exempt de sérum, contenant en solution un polymère agissant comme protecteur des cellules par réduction du drainage filmogène autour des cellules cultivées en réacteurs agités.

Les polymères utilisés à cet effet sont les polyéthylèneglycols, la polyvinylpyrrolidone et les polymères contenant un ou plusieurs groupes oxyde d'alkylène.
- la demande de brevet EP 14.519 qui préconise l'utilisation de polyéthylèneglycol comme agent de fusion pour la fabrication d'hybridomes.
- le Derwent abstracts n° 87-133257/19 qui préconise la culture de cellules animales à haute densité en présence de polyglycol.

Au niveau industriel, il est plus intéressant d'augmenter la productivité spécifique des cellules plutôt que d'améliorer leur croissance, de manière à obtenir des rendements élevés en protéines sans mettre en oeuvre des quantités importantes de cellules. D'autre part, il est souhaitable de disposer de milieux de culture exempts de sérum.

On a maintenant trouvé que la polyvinylpyrrolidone (ci-après parfois appelée PVP) pouvait être utilisée comme agent activateur de la productivité spécifique des cellules animales et permettait l'obtention de milieux de culture définis pour des cellules animales, en particulier :
- les cellules animales recombinantes, par exemple les cellules d'ovaire de hamster chinois (Chinese Hamster Ovary cells), dénommées ci-après cellules CHO, productrices d'interleukine-2 (IL$_2$) ou d'antigène de surface du virus de l'hépatite B, et les cellules de reins de singe vert d'Afrique, appelées ci-après cellules Vero, productrices d'hormone de croissance humaine (hGH),
- les hybridomes, par exemple les hybridomes secréteurs d'anticorps.

Les cellules CHO sont des cellule animales de choix pour la production de protéines par voie génétique.

Elles sont généralement cultivées sur des milieux de culture constitués du milieu MEM (milieu de base minimum) complémenté de 5 % de sérum de veau foetal.

La productivité spécifique est définie comme la production en protéines par unité de temps et par unité de biomasse, cette dernière unité étant exprimée dans la présente description en unité de densité optique (uDO).

L'agent activateur de productivité spécifique selon l'invention contient donc de la polyvinylpyrrolidone.

On peut utiliser selon l'invention les polyvinylpyrrolidones ayant un poids moléculaire moyen d'au moins 2500 et de préférence compris entre 2 500 et 1 200 000, en particulier entre 2 500 et 750 000. Dans le cas où la polyvinylpyrrolidone est sous forme solide (par exemple la polyvinylpyrrolidone de poids moléculaire moyen de 1 200 000) on utilisera cette dernière en suspension dans un milieux aqueux. Les polyvinylpyrrolidones préférées aux fins de l'invention sont celles ayant un poids moléculaire moyen de 40.000 environ.

L'agent activateur selon l'invention peut en outre contenir un copolymère d'oxyde d'éthylène et d'oxyde de propylène de poids moléculaire moyen calculé à partir de l'indice d'OH, compris entre 1100 et 8500 environ.

Des exemples de copolymères d'oxyde d'éthylène et d'oxyde de propylène appropriés aux fins de l'invention sont ceux connus sous la dénomination commerciale PLURIOL (BASF). Le PLURIOL PE 6800 de poids moléculaire moyen de 8500 est particulièrement préféré aux fins de l'invention.

La teneur en copolymère d'oxyde d'éthylène et d'oxyde de propylène doit représenter environ 0,5 à 50 % de la teneur en polyvinylpyrrolidone.

L'invention concerne également les milieux de culture définis pour les cellules animales contenant l'agent activateur de productivité spécifique selon l'invention ; ce milieu permet d'augmenter la productivité spécifique des cellules animales et plus particulièrement des cellules animales recombinantes, notamment les cellules CHO et les cellules Vero, et des hybridomes tout en minimisant la croissance de celles-ci. Il comprend :
- un milieu de base défini ;
- un inducteur de croissance,
- un agent activateur de la productivité spécifique contenant une polyvinylpyrrolidone, la teneur globale en inducteur de croissance dans ledit milieu n'excédant pas 50 mg/l et la teneur en agent activateur de productivité spécifique étant comprise entre 0,1 et 10 % en poids par rapport au volume du milieu. On désigne par "% en poids par rapport au volume du milieu" des grammes/100 ml de milieu.

Il est connu que, de manière générale, on prépare un milieu de base défini en mélangeant au moins deux milieux de base classiques.

Des exemples de milieux de base qui peuvent être utilisés selon la présente invention comprennent notamment les différents milieux de base disponibles dans le commerce, par exemple :
- le milieu minimum essentiel de EAGLE (MEM) (Science, 130, 432, 1959) ;
- le milieu de base EAGLE (BME) (Proceedings of the Society for Experimental Biology and Medicine, 89, 362, 1965) ;
- le milieu de EAGLE modifié par DULBECCO (DME) (Virology, 8, 396, 1959)
- le milieu de DULBECCO modifié par ISKO (IMDM) (The Journal of Experimental Medicine, 147, 923, 1978)
- le milieu L-15 (Proceedings of the Society for Experimental Biology and Medicine, 100, 115, 1959)
- le milieu F 12 de HAM (Proceedings of National Academy of Science, USA, 53, 288, 1965)

- le milieu RPMI 1640 (Journal of the American Medical Association, 199, 519, 1967) ou un mélange de ces milieux.

De manière avantageuse, le mélange de milieux de base est complémenté en oligoéléments et en acides aminés non essentiels. Il appartiendra à l'homme de l'art de sélectionner par des essais de routine, les oligoéléments et les acides aminés appropriés ainsi que leurs proportions relatives en fonction des milieux de base mis en oeuvre et des cellules à cultiver.

L'inducteur de croissance du milieu de culture selon l'invention est une protéine telle que l'insuline qui favorise l'initiation de la croissance; celle-ci peut être avantageusement utilisée en combinaison avec un transporteur du fer, tel que par exemple la transferrine en présence de fer ou avantageusement utilisée en combinaison avec un complexe de fer, tel que le ferricitrate.

Alors que dans les milieux de culture classiques complémentés avec du sérum, la quantité de protéines à mettre en oeuvre est d'environ 2 g/l de milieu, on notera que dans le milieu de culture défini selon l'invention, la teneur globale en substances protéiques ne doit pas dépasser environ 50 mg/l. Elle est de préférence comprise entre environ 3 mg/l et 35 mg/l.

En effet au delà de cette teneur, l'activité cellulaire décroit ainsi que la productivité spécifique.

Par contre, pour des teneurs en substances protéiques inférieures à 50 mg/l, la productivité spécifique est considérablement accrue même si la croissance cellulaire est diminuée.

Selon une variante préférée de l'invention, le milieu de culture comprend en poids par rapport au volume du milieu, 3 à 6 % de PVP ayant un poids moléculaire de 40.000 et éventuellement 0,5 ‰ de PLURIOL PE 6800 de poids moléculaire moyen de 8500.

De plus, le milieu de culture selon l'invention est tamponné à pH 7,2 à l'aide d'un tampon approprié, tel que les tampons MOPS (acide 3-[N-Morpholino-]propanesulfonique) ou HEPES (acide N- [2-hydroxyéthyl] piperazine-N'- [2-éthanesulfonique)] (commercialisés par SIGMA).

Le milieu de culture selon l'invention est particulièrement approprié pour la production de $IL_2$ par des cellules CHO transfectées ; il permet en effet d'augmenter la production totale en $IL_2$ par exemple d'un facteur 1,5 et d'augmenter la productivité spécifique par exemple d'un facteur d'au moins 2.

Il est avantageux que le milieu de culture selon l'invention contienne en outre du fer sous forme ionique, par exemple du sulfate de fer, et un transporteur de fer, tel que la transferrine, ou un complexe de fer, tel que le ferricitrate.

L'invention concerne également un procédé de production d'un métabolite d'intérêt consistant à mettre en culture des cellules animales susceptibles de produire ce métabolite dans le milieu de culture précédemment défini. Ce procédé peut être mis en oeuvre dans les réacteurs habituels des procédés de culture des cellules animales.

La présente invention va être maintenant décrite en détail par les exemples illustratifs mais non limitatifs ci-après.

EXEMPLE 1 PRODUCTION D'IL₂ DANS LES CELLULES CHO EN REACTEUR AGITE.

Dans cet exemple on a cultivé des cellules CHO DX BII (clone de CHO K₁ - DHFR⁻ décrit par URLAUB et al. dans Proc. Natl. Acad. Sci. USA Vol. 77, n° 7, pp. 4216-4220, Juillet 1980), transfectées par le plasmide recombinant codant pour la protéine IL₂ humaine glycosylée décrit par FERRARA et al. dans FEBS Letters Vol. 226, n° 1, p. 47-52, 1987.

1) dans un milieu de culture défini selon l'invention constitué d'un milieu de base défini comprenant un mélange de deux milieux de base MEM et F12 complémenté par un stock d'oligoéléments et d'acides aminés non essentiels et contenant de l'insuline (I), de la transferrine (T), de la polyvinylpyrrolidone et éventuellement du PLURIOL PE 6800 (BASF), copolymère d'oxyde d'éthylène et d'oxyde de propylène d'un poids moléculaire moyen de 8500. La composition de ce milieu est donnée dans les tableaux ci-après;

2) dans un milieu témoin à des fins de comparaison.

Ce milieu témoin comprend un milieu de base défini (mélange des milieux de base MEM et F12) complémenté par un stock d'oligoéléments et d'acides aminés non essentiels auquel on ajoute 500 mg/l d'une fraction de sérum. Il s'agit donc d'un milieu non défini. Cette fraction correspond à la fraction IV.1 décrite par COHN et al. dans J. Am. Chem. Soc. 1946, 68, 459. Ce substitut de sérum permet une croissance et une production équivalente à celles obtenues avec un milieu complémenté en sérum pour une cellule d'hybridome de souris ES-6 (MacLeod et al. Develop. Biol. Standard, Vol. 60 p.55-61 - S.Karger, Basel, 1985).

Ce milieu témoin dénommé ci-après milieu MEM/F12 FIV.1, permet de diminuer le contenu protéique d'un facteur quatre par rapport à un milieu classique contenant 5 % de sérum de veau foetal.

On notera que le milieu de repiquage ou de stockage des cellules, utilisé au cours de ces essais est un milieu de base minimum MEM contenant 5 % de sérum de veau foetal.

Les cultures ont été réalisées en situation de procédé en réacteur agité (dit réacteur spinner) d'un volume utile de 100 ml, par ensemencement de 3.10⁵ cellules par millilitre au départ. La culture dure 16 jours en présence de microporteurs (cytodex 3) à 5 g/l, avec un renouvellement du milieu du réacteur par jour, et sous agitation (30t/min) dans une chambre chaude à 37° C.

Au cours de ces essais on a utilisé comme mode d'analyse, le comptage cellulaire en culture sur microporteurs par coloration au cristal violet et comptage des noyaux cellulaires sur cellules de NEUBAUER.

Le dosage de IL₂ dans le milieu de culture a été réalisé par mesure de l'activité biologique du milieu de culture selon le test colorimétrique de Mosmann, T. (1983) J. Immunol. Methods, 65, 55-63 - sur la prolifération de la lignée de lymphocytes-T de souris CTLL-2 dépendante de l'IL₂ (Baker, P. et al (1979) J.

Exp. Med., 149, 173). A titre de témoin, la préparation de référence décrite dans Lymphokine Research ((1984), 4, 193-227) a été utilisée.

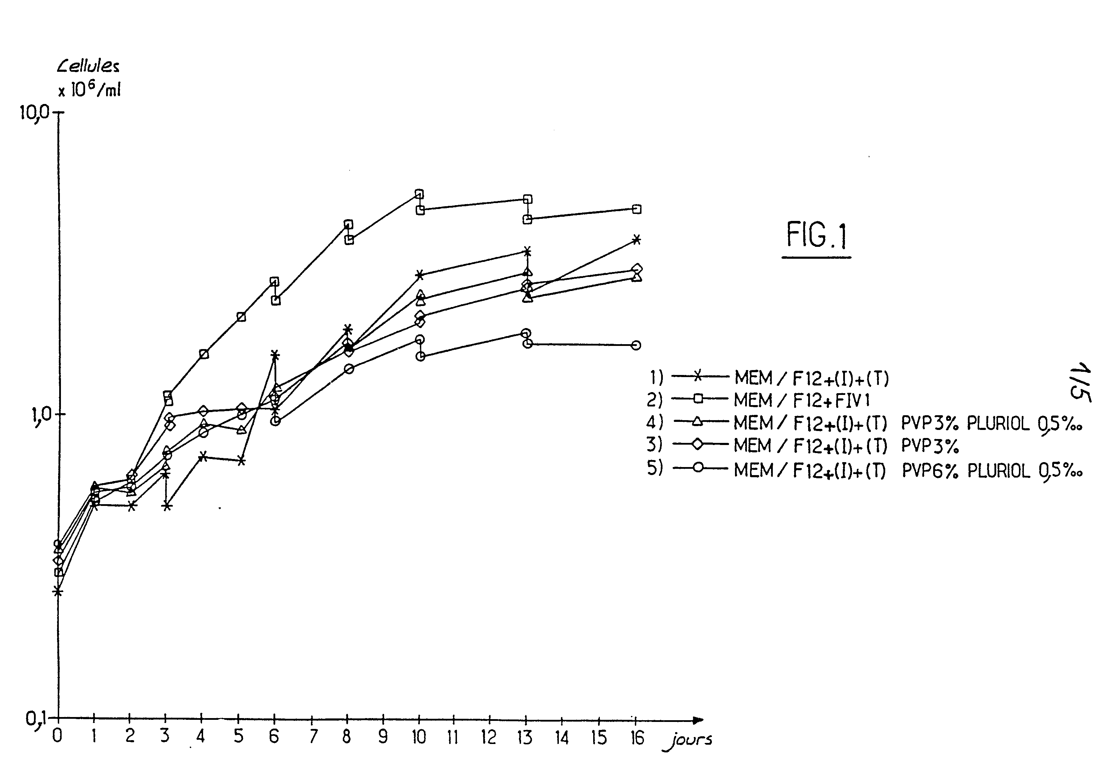

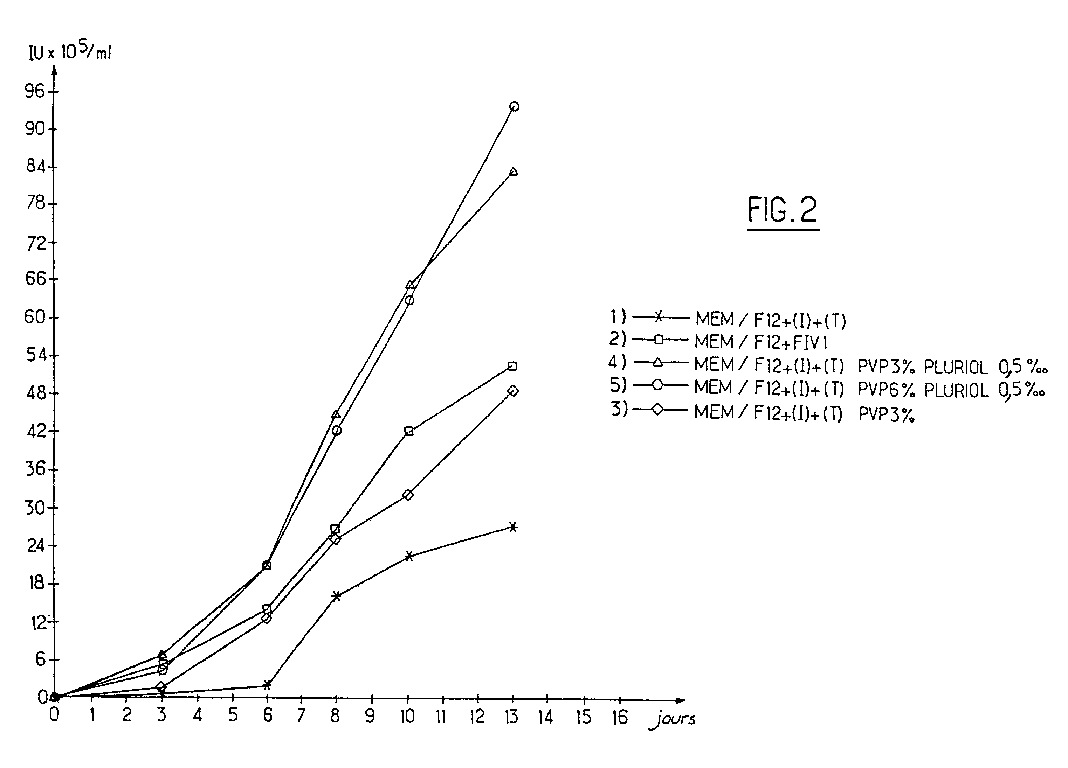

Les résultats obtenus sont reportés sur les figures 1 et 2 annexées sur lesquelles:

-La Figure 1 représente les courbes de croissance des cellules obtenues en réacteur avec les milieux ci-après:

| 1) milieu sans PVP | : MEM/F12 + (I) + (T) |
|---|---|
| 2) milieu témoin | : MEM/F12 + F IV.1 |
| 3) milieu avec PVP | : MEM/F12 + (I) + (T) + PVP 40 à 3 % |
| 4) milieu avec PVP + PLURIOL | : MEM/F12 + (I) + (T) + PVP 40 à 3% + PLURIOL PE 6800 à 0,5 %₀ |
| 5) milieu avec PVP + PLURIOL | : MEM/F12 + (I) + (T) + PVP 40 à 6 % + PLURIOL PE 6800 à 0,5 %₀. |

Le PVP mis en oeuvre dans ces essais est du PVP 40, c'est-à-dire un PVP ayant un poids moléculaire moyen de 40.000.

Ces courbes de croissance (nombre de cellules/ ml) en fonction du temps en jours) montrent que :
- plus la concentration de PVP est forte, plus la croissance est limitée.
- les milieux 1, 3 et 4 ont à peu près les mêmes caractéristiques de croissance; L'adjonction de PVP à 3 % et de PLURIOL modifie donc peu l'allure de la courbe de croissance (3.10⁶ cellules/ml). On peut voir une légère diminution de la croissance.
- le milieu témoin présente une meilleure capacité de croissance (5.10⁶ cellules/ml).

La Figure 2 donne les courbes de production en IL-2 exprimée en unités internationales par ml en fonction du temps exprimé en jours. On peut constater en observant cette figure que :
- la production cumulée en IL₂ est du même ordre de grandeur entre le milieu témoin 2 et le milieu 3, alors que le milieu 1 présente deux fois moins de production cumulée. L'adjonction de PVP à un milieu défini permet donc d'augmenter la production cumulée en IL₂ par un facteur voisin de 2, laquelle est alors équivalente à celle d'un milieu non défini. La productivité spécifique moyenne (exprimée en unités internationales d'IL₂/10⁶ cellules/24 heures), calculée sur l'ensemble des expériences correspondant au milieu 3 est augmentée d'un facteur 1,3 par rapport à cette grandeur pour le milieu 1. Le PVP est donc un agent activateur de la productivité spécifique en IL₂ des cellules CHO.
- l'adjonction de PLURIOL à 0,5 %₀ (milieu 4) permet de dépasser la production en IL₂ du milieu témoin (milieu 2) sans toucher à la croissance. La productivité spécifique moyenne calculée sur l'ensemble des expériences est alors augmentée d'un facteur 3 par rapport au milieu défini seul (milieu 1).
- la productivité spécifique maximale est obtenue avec la plus forte concentration en PVP (milieu 5 contenant 6 % de PVP) puisque l'on a la meilleure production cumulée de produit pour une croissance très ralentie.

La présence de PVP dans le milieu défini selon l'invention permet, même s'il limite la croissance, d'augmenter la productivité spécifique d'IL$_2$. La présence de PLURIOL amplifie ce phénomène pour les plus faibles concentrations en PVP.

EXEMPLE 2 : PRODUCTION D'HORMONE DE CROISSANCE HUMAINE (hGH) ET D'ANTIGENE DE SURFACE DU VIRUS DE L'HEPATITE B (HBS) DANS LES CELLULES CHO, EN CULTURE STATIQUE.

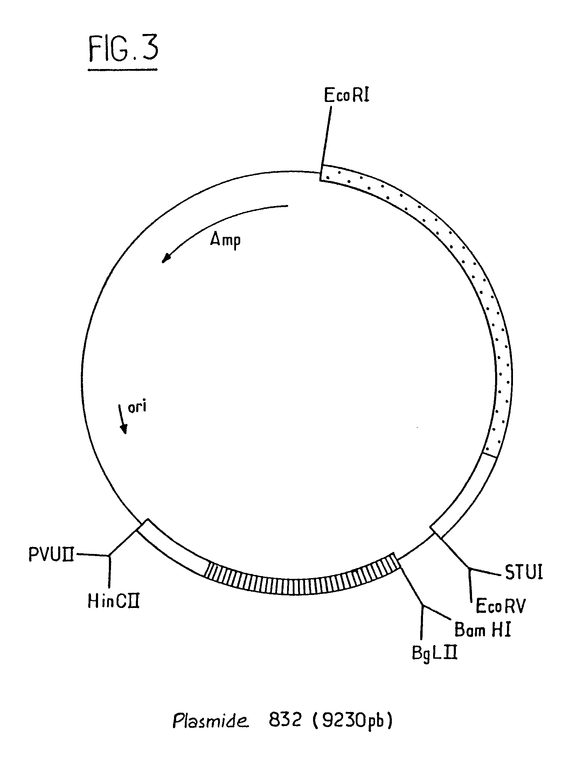

Dans cet exemple, on a cultivé des cellules CHO DX BII (référence déjà citée) :
* soit transfectées par le plasmide recombinant décrit dans l'exemple 1 mais dans lequel l'ADN$_C$ de l'IL$_2$ humaine a été remplacé par l'ADN$_C$ de l'hGH (décrit par ROSKAM et ROUGEON dans Nucl. Acids Res. 7, 1979, - p. 305-320).
* soit transfectées par le plasmide 832 de 9230 paires de bases, dont la carte de restriction est représentée sur la figure 3.

Ce plasmide comprend :
- un fragment EcoRI - PVU II de pBR 322 (représentée par

————————)

de 2295 paires de bases contenant l'origine de réplication bactérienne (ori) et le gène de résistance à l'ampicilline (Amp).
- un fragment Hinc II - Bgl II de 3600 paires de bases contenant l'unité d'expression pour l'antigène de surface de l'hépatite B de sous type ADW (décrit par ONO et al., dans Nucleic Acids Research 11, 1983, 1474-1757).

Cette unité d'expression comprend :
- le promoteur MTH$_1$ de souris (représenté par

[▭]   )

) décrit par BRINSTER (BRINSTER et al, Nature 296, 1982, 39-42).
- les séquences codantes pour les régions pré-S$_2$, S et 3' non traduites du virus de l'hépatite B (représentées par

[▥]   ).

). - un fragment Bam HI-EcoRV de pBR 322 de 190 paires de bases (représenté par

——————— ).

). - un fragment STUI-EcoRI de 3145 paires de bases contenant l'unité d'expression pour la DHFR.

Cette unité d'expression comprend :
- le promoteur MTH1 constitutif décrit par STUART (STUART et al., PNAS USA 81, 1984, 7318-7322) (représenté par

[▭]   ).

) - la séquence codante pour la DHFR de souris et l'intron de l'antigène t du virus SV40 ainsi que le signal précoce de polyadénylation de ce même virus (représenté par

[▱]  ).

).

Ces cellules ont été cultivées :
1) dans le milieu de culture défini selon l'invention
2) dans le milieu témoin non défini comme mentionné dans l'exemple 1.

Les cultures ont été réalisées avec des plaques à 24 puits pour culture cellulaire (commercialisées par Falcon), par ensemencement de $3.10^4$ cellules par puits dans un volume utile de 1 ml. La culture dure 7 jours dans un incubateur à 37°C régulé à 5 % de $CO_2$ avec renouvellement du milieu de culture au bout de 4 jours.

Au cours de ces essais, on a utilisé comme mode d'analyse :
- l'évaluation de la croissance cellulaire selon le test colorimétrique de Mosmann T. décrit dans (1983) J. Immunol. Methods 65, 55-63 ;
- le dosage de l'hGH selon une méthode radioimmunologique grâce à un kit "COATRIA" commercialisé par BIO-MERIEUX. Ce dosage repose sur une double réaction immunologique effectuée dans des tubes à essais "COATRIA". Cette dernière est une réaction d'inhibition compétitive utilisant un anticorps Ab$_2$ dirigé contre un anticorps Ab$_1$ anti-hGH, de l'hGH radiomarqué à l'Iode [125] et l'hGH de l'échantillon à doser ou l'hGH de référence ;
- le dosage de l'HBS selon une méthode également radioimmunologique grâce à un kit AUSRIA II commercialisé par ABBOTT. Ce dosage repose sur une double réaction immunologique de type sandwich utilisant un premier anticorps Ab$_1$ dirigé contre l'HBS, un deuxième anticorps Ab$_2$ radiomarqué, lui aussi dirigé contre l'HBS et l'échantillon à doser ou le témoin.

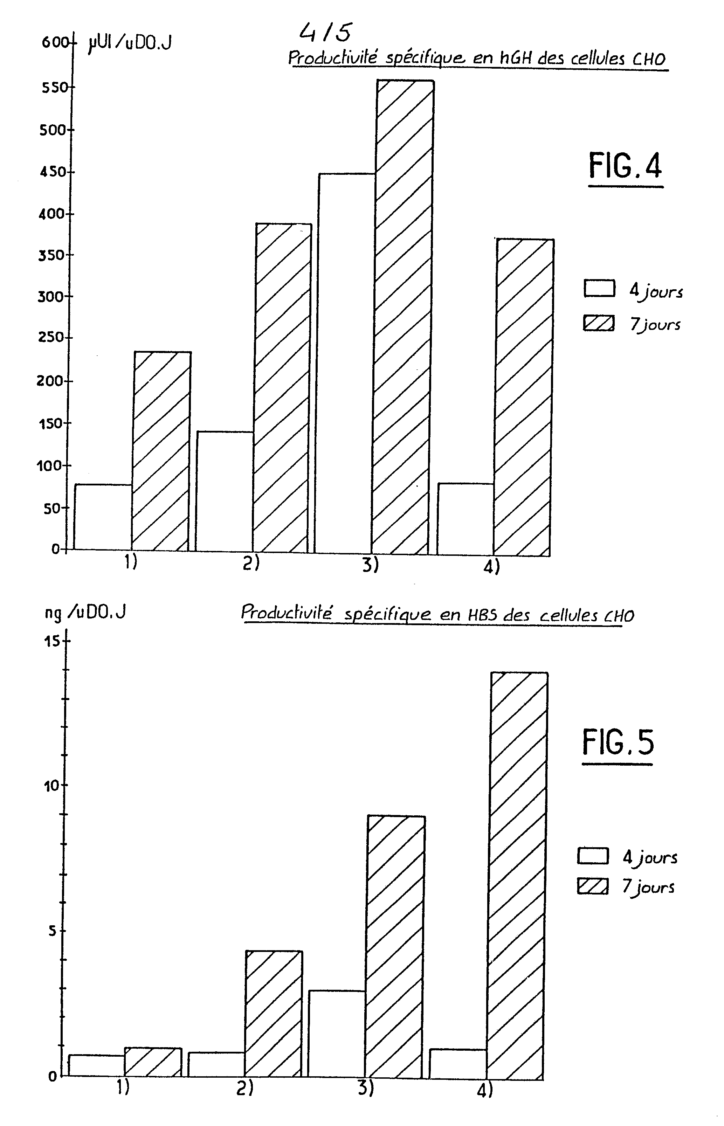

Les résultats obtenus sont présentés sur les figures 4 et 5 annexées sur lesquelles :
La figure 4 représente sous forme d'histogramme les productivités spécifiques des cellules CHO productrices d'hGH à 4 et 7 jours de culture (exprimées en mic-unités internationales/uDO.J) avec les milieux ci-après :

1) milieu sans PVP : MEM/F12 + (I) + (T)
2) milieu avec PVP : MEM/F12 + (I) + (T) + PVP40 à 3 %
3) milieu avec PVP + PLURIOL : MEM/F12 + (I) + (T) + PVP40 à 3 % + PLURIOL PE 6800 à 0,5 %o
4) milieu témoin : MEM/F12 + FIV.1

La figure 5 représente sous forme d'histogramme les productivités spécifiques des cellules CHO productrices d'HBS à 4 et 7 jours de culture (exprimées en nanogrammes/unité DO.J) avec les milieux ci-après :

1) milieu sans PVP : MEM/F12 + (I) + (T)
2) milieu avec PVP : MEM/F12 + (I) + (T) + PVP40 à 3 %
3) milieu avec PVP + PLURIOL : MEM/F12 + (I) + (T) + PVP40 à 3 % + PLURIOL PE 6800 à 0,5 %o
4) milieu témoin : MEM/F12 + FIV.1

Le PVP mis en oeuvre dans ces essais est du PVP40, c'est-à-dire un PVP ayant un poids moléculaire moyen de 40 000. On constate que :
- la productivité spécifique est augmentée par un facteur au minimum égal à 1,25 quand on ajoute du PVP à 3 % dans le milieu de base.
- pour l'hGH l'adjonction de PLURIOL à 0,5 %o permet d'augmenter la productivité spécifique d'un facteur au moins égal à 3 par rapport au milieu défini seul et de dépasser la productivité spécifique du milieu témoin.

Pour HBS, le milieu PVP PLURIOL permet de produire neuf fois plus par unité de DO et par unité de temps que le milieu de base sans cependant rattraper la productivité spécifique du milieu témoin.

EXEMPLE 3 : PRODUCTION D'hGH DANS LES CELLULES VERO EN CULTURE STATIQUE

Dans cet exemple, on a cultivé des cellules Véro (décrites par YASUMURA Y. et al. dans NIPPON Rinsho 21, p. 1209, 1963) transfectées par le plasmide recombinant codant pour la protéine hGH décrit par LUPKER et al. dans GENE 24, p. 281-287, 1983.

    1) dans un milieu de culture défini selon l'invention
    2) dans un milieu témoin non défini à des fins de comparaison.

On notera que le milieu de repiquage ou de stockage des cellules, utilisé au cours de ces essais est un milieu de base minimum MEM contenant 5 % de sérum de veau foetal.

Les cultures ont été réalisées avec des plaques à 24 puits comme dans l'exemple 2.

Au cours de ces essais, on a utilisé comme mode d'analyse:
- l'évaluation de la croissance cellulaire selon le test mentionné dans l'exemple 2 ;
- le dosage de l'hGH selon une méthode radioimmunologique grâce à un kit "COATRIA" commercialisé par BIO-MERIEUX (cf. exemple 2).

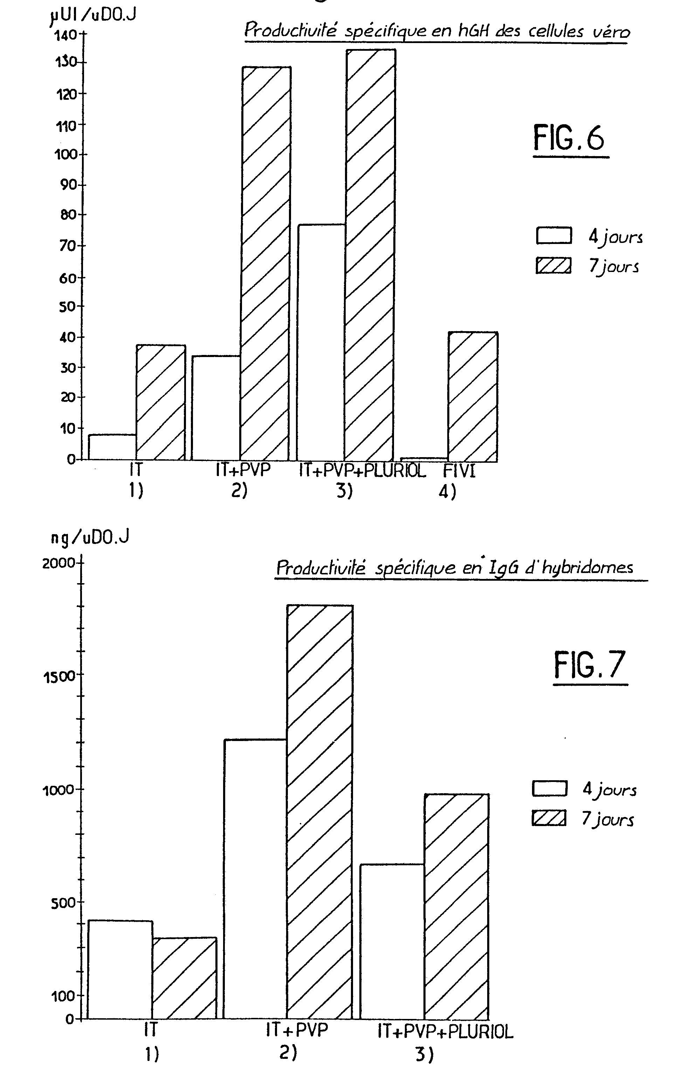

Les résultats obtenus sont reportés sur la figure 6 annexée représentant sous forme d'histogramme les productivités spécifiques des cellules Vero productrices d'hGH à 4 et 7 jours de culture (exprimées en microunités internationales/ uDO.J) avec les milieux ci-après :

1) milieu sans PVP : MEM/F12 + (I) + (T)
2) milieu avec PVP : MEM/F12 + (I) + (T) + PVP40 à 3 %
3) milieu avec PVP + PLURIOL : MEM/F12 + (I) + (T) + PVP40 à 3 % + PLURIOL PE 6800 à 0,5 %o
4) milieu témoin : MEM/F12 + FIV.1

Le PVP mis en oeuvre dans ces essais est du PVP40 c'est-à-dire du PVP ayant un poids moléculaire moyen de 40000. On constate que :
- la productivité spécifique est augmentée par un facteur au minimum égal à 1,3 en utilisant du PVP à 3 % dans le milieu de base défini ;
- l'adjonction de PLURIOL à 0,5 %o permet d'augmenter la productivité spécifique d'un facteur au moins égal à 3, jusqu'à dépasser celle du milieu témoin non défini.

Exemple 4 : PRODUCTION D'ANTICORPS MONOCLONAUX PAR DES HYBRIDOMES EN CULTURE STATIQUE

Dans cet exemple, on a cultivé les hybridomes F 93609 issus de la fusion de cellules de myélome murin (NS2) (sous-clone du myélome murin P3X$_{63}$Ag8 déposé à l'ATCC sous le No. CRL 1580 et décrit dans J. of Immunology, 123, p. 1548-1550 (1979) ) et de lymphocytes de souris femelles Balb C.

La fusion a été effectuée selon la méthode décrite par KOHLER et MILSTEIN dans nature 256 (1975) 495, résumée ci-après :

Des souris Balb C. ont été immunisées par injection périodique d'antigènes. A l'issue de l'immunisation, les cellules de la rate, parmi lesquelles se trouvent les cellules secrétant les anticorps attendus, sont isolées et fusionnées avec les cellules de myélome. La fusion est réalisée en présence de polyéthylène-glycol (PEG) ; le produit de cette fusion est l'hybridome.

Cet hybridome secrète des anticorps monoclonaux ST1 de classe IgG 2A, anticorps reconnaissant l'antigène membranaire T 65 des cellules T mature.

On a cultivé cet hybridome :
    1) dans un milieu de culture défini selon l'invention;
    2) dans un milieu témoin à des fins de comparaison.

On notera que le milieu de repiquage ou de stockage des cellules, utilisé au cours de cet essai, est un milieu RPMI 1640 (GIBCO) contenant 5 % de sérum de veau foetal.

Les cultures ont été réalisées en plaque à 24 puits, comme dans l'exemple 2.

Au cours de cet essai, on a utilisé comme mode d'analyse :
- l'évaluation de la croissance cellulaire, selon le test mentionné dans l'exemple 2 ;
- le dosage des IgG, selon une méthode immunoenzymatique grâce à un test Elisa, commercialisé par Biosys (référence BI 9013 pour dosage des IgG de souris). Cette méthode repose sur un dosage immunologique par la méthode dite sandwich : elle utilise les anticorps de mouton anti-IgG de souris marqué à la phosphatase alcaline, un anticorps adsorbé sur plaque dirigé contre les immunoglobulines de souris, et l'échantillon à doser ou le témoin.

Les résultats obtenus sont reportés sur la figure 7, annexée, représentant sous forme d'histogramme les productivités spécifiques des hybridomes secréteurs d'IgG, à 4 et 7 jours de culture (exprimées en nanogrammes/unité DO.J) avec les milieux ci-après :

| | |
|---|---|
| 1) milieu sans PVP | : MEM/F12 + (I) + (T) |
| 2) milieu avec PVP | : MEM/F12 + (I) + (T) + PVP40 à 3 % |
| 3) milieu avec PVP + PLURIOL | : MEM/F12 + (I) + (T) + PVP40 à 3 % + PLURIOL PE 6800 à 0,5 ‰ |

Le PVP mis en oeuvre dans ces essais est du PVP40 c'est-à-dire du PVP ayant un poids moléculaire moyen de 40 000. On constate que :
- la productivité spécifique est augmentée par un facteur, au minimum égal à 3, en utilisant du PVP 3 % dans le milieu défini ;
- l'adjonction du PLURIOL à 0,5 ‰ n'a pas, dans cet essai, d'effet synergique avec le PVP.

La présence de PVP, dans le milieu défini selon l'invention, permet donc même s'il limite la croissance, d'augmenter la productivité spécifique de nombreuses cellules productrices de protéines d'intérêt.

Des résultats similaires sont obtenus en remplaçant dans le milieu défini, dont la composition est donnée ci-dessus, la transferrine et le sulfate de fer par un complexe de fer tel que le ferricitrate à la molarité de $10^{-5}$ M.

TABLEAU I

**CONSTITUTION DU MILIEU DEFINI COMPLET**

| ACIDES AMINES | PM | mg/l | mM |
|---|---|---|---|
| L- Alanine | 89,09 | 13,35 | 0,15 |
| L- Arginine. HCl | 210,7 | 168,5 | 0,8 |
| L-Asparagine. H₂O | 150 | 22,5 | 0,15 |
| L-Cystine. 2HCl | 313,2 | 15,65 | 0,05 |
| L- Cysteine. HCl. H₂O | 175,6 | 17,55 | 0,10 |
| L- acide glutamique | 147 | 22,05 | 0,15 |
| L- Glutamine | 146 | 292 | 2 |
| L- Glycine | 75,07 | 11,25 | 0,15 |
| L- Histidine. HCl. H₂O | 209,7 | 31,48 | 0,15 |
| L- Isoleucine | 131,2 | 27,97 | 0,21 |
| L- Leucine | 131,2 | 32,55 | 0,25 |
| L- Lysine. HCl | 182,7 | 54,5 | 0,30 |
| L-Methionine | 149,2 | 9,74 | 0,065 |
| L-Phenylalanine | 165,2 | 18,5 | 0,11 |
| L- Proline | 115 | 28,75 | 0,25 |
| L- Serine | 105 | 15,75 | 0,15 |
| L- Threonine | 119,1 | 30 | 0,25 |
| L- Tyrosine | 181,2 | 29,89 | 0,165 |
| L-Tryptophane | 204,2 | 6 | 0,03 |
| L- Valine | 117,2 | 28,85 | 0,246 |
| L- acide aspartique | 133 | 19,95 | 0,15 |

| VITAMINES | PM | mg/l | mM |
|---|---|---|---|
| Biotine | 244,3 | $3,65\ 10^{-3}$ | $0,015\ 10^{-3}$ |
| Chlorure de choline | 139,6 | 7,48 | 0,05 |
| Acide folique | 441,4 | 2,45 | 0,003 |
| Nicotinamide | 122,13 | 0,513 | $4,6\ 10^{-3}$ |
| Pyridoxal. HCl | 203,63 | 0,5 | $2,5\ 10^{-3}$ |
| Pyridoxine. HCl | 205,6 | 0,031 | $3\ 10^{-4}$ |
| Riboflavine | 376,4 | 0,069 | $2\ 10^{-4}$ |
| Thiamine. HCl | 337,3 | 0,67 | $2\ 10^{-3}$ |
| Cyanocoba-lamine | 1355,4 | 0,68 | $5\ 10^{-4}$ |
| Pantothe-nate de calcium D | 238,3 | 0,74 | $3\ 10^{-3}$ |

| SELS INORGANI-QUES | PM | mg/l | mM |
|---|---|---|---|
| $CaCl_2$ | 110,99 | 86,5 | 0,73 |
| KCl | 74,56 | 311,8 | 4,18 |
| $KH_2\ PO_4$ | 136,09 | 30 | 0,22 |
| $FeSO_4.\ 7H_2O$ | 278,02 | 1,8 | $6,5\ 10^{-3}$ |
| $MgSO_4$ | 120,37 | 48,84 | 0,4 |
| NaCl | 58,44 | 7800 | 133 |
| $NaHCO_3$ | 84,01 | 350 | 4,1 |
| $MgCl_2$ | 95,22 | 28,5 | 0,3 |
| $Na_2HPO_4$ | 141,96 | 94,94 | 0,67 |

| OLIGOELEMENTS | PM | mg/l | mM |
|---|---|---|---|
| $CuSO_4.\ 5H_2O$ | 249,68 | $1,5\ 10^{-3}$ | $6\ 10^{-6}$ |
| $MnSO_4.\ H_2O$ | 169,02 | $0,845\ 10^{-3}$ | $5\ 10^{-6}$ |
| $Na_2SeO_3.\ 5H_2O$ | 263,01 | $3,42\ 10^{-2}$ | $1,4\ 10^{-4}$ |
| $NiCl_2.\ 6H_2O$ | 237,70 | $1,19\ 10^{-6}$ | $5\ 10^{-9}$ |
| $(NH_4)_6\ Mo_7O_{24}.\ 4H_2O$ | 1235,89 | $1,235\ 10^{-3}$ | $1\ 10^{-6}$ |
| $NH_4\ VO_3$ | 116,99 | $5,845\ 10^{-4}$ | $5\ 10^{-6}$ |
| $SiO_2\ Na_2O.\ 5H_2O$ | 212,14 | 0,1067 | $5\ 10^{-4}$ |
| $SnCl_2.\ 2H_2O$ | 225,63 | $1,12\ 10^{-6}$ | $5\ 10^{-9}$ |
| $ZnSO_4.\ 7H_2O$ | 287,54 | 2,13 | $7\ 10^{-3}$ |

| AUTRES CONSTI-TUANTS | PM | mg/l | mM |
|---|---|---|---|
| D. Glucose | 18 | 1800 | 10 |
| Rouge de phénol (sel sodique) | | 11 | |
| Pyruvate de Sodium | 110 | 110 | 1 |
| Hypoxanthine (sel sodique) | 136,1 | 2,38 | $1,75\ 10^{-2}$ |
| Acide lipoique | 206,3 | 0,105 | $5\ 10^{-4}$ |
| Acide Linoléique | 278,44 | 0,042 | $1,5\ 10^{-4}$ |
| Putrescine 2HCl | 161,1 | 0,0805 | $5\ 10^{-4}$ |
| i-Inositol | 180,2 | 10 | $5,5\ 10^{-2}$ |
| Thymidine | 242,2 | 0,365 | $1,5\ 10^{-3}$ |

| ADJUVANTS | PM | mg/l | mM |
|---|---|---|---|
| MOPS | 209,3 | 6279 | 30 |
| Insuline | | entre 3 et10 | |
| Transferrine | | entre 1 et25 | |
| Pluriol 6800 | | 500 | |
| PVP (*) | | 30000 ou 60000 | |

(*) PVP 2500, 10.000, 20.000, 40.000 et 750.000.

## Revendications

1. Agent activateur de productivité spécifique de cellules animales caractérisé en ce qu'il contient une polyvinylpyrrolidone ayant un poids moléculaire moyen d'au moins 2500.

2. Agent activateur selon la revendication 1, caractérisé en ce que les cellules animales sont des cellules animales recombinantes.

3. Agent activateur selon la revendication 2, caractérisé en ce que les cellules animales recombinantes sont des cellules CHO.

4. Agent activateur selon la revendication 2, caractérisé en ce que les cellules animales recombinantes sont des cellules Vero.

5. Agent activateur selon l'une quelconque des revendications 1 à 4, caractérisé en ce que la polyvinylpyrrolidone a un poids moléculaire moyen compris entre 2500 et 1.200.000, de préférence entre 2500 et 750.000.

6. Agent activateur selon l'une quelconque des revendications 1 à 5 , caractérisé en ce qu'il contient en outre un copolymère d'oxyde d'éthylène et d'oxyde de propylène de poids moléculaire moyen, calculé à partir de l'indice d'OH, compris entre 1100 et 8500 environ.

7. Agent activateur selon la revendication 6, caractérisé en ce que le copolymère d'oxyde d'éthylène et d'oxyde de propylène représente environ 0,5 à 50 % de la teneur en polyvinylpyrrolidone.

8. Milieu de culture défini pour cellules animales caractérisé en ce qu'il comprend :- un milieu de base défini ;
- un inducteur de croissance,
- un agent activateur de productivité spécifique selon l'une quelconque des revendications 1 à 7, la teneur globale en inducteur de croissance dans ledit milieu n'excédant pas 50 mg/l et la teneur en agent activateur de productivité spécifique étant comprise entre 0,1 et 10 % en poids par rapport au poids du volume du milieu.

9. Milieu de culture défini selon la revendication 8, caractérisé en ce qu'il comprend en outre des oligoéléments et des acides aminés non essentiels.

10. Milieu de culture selon l'une des revendications 8 et 9, caractérisé en ce qu'il contient en poids par rapport au volume du milieu, de 3 à 6 % d'une polyvinylpyrrollidone ayant un poids moléculaire moyen de 40.000.

11. Milieu de culture selon la revendication 10, caractérisé en ce qu'il contient en outre, en poids par rapport au volume du milieu, 0,5 ‰ d'un copolymère d'oxyde d'éthylène et d'oxyde de propylène ayant un poids moléculaire moyen de 8.500.

12. Milieu de culture selon l'une des revendications 10 et 11, caractérisé en ce qu'il est tamponné à pH 7.2.

13. Milieu de culture selon l'une quelconque des revendications 8 à 12, caractérisé en ce que l'inducteur de croissance est l'insuline.

14. Milieu de culture selon l'une des revendications 8 à 13, caractérisé en ce qu'il comprend en outre du fer sous forme ionique et un transporteur de fer, tel que la transferrine, ou un complexe de fer tel que le ferricitrate.

15. Procédé de production d'un métabolite consistant à mettre en culture des cellules animales susceptibles de produire ce métabolite dans un milieu de culture selon l'une des revendications 8 à 14.

16. Procédé selon la revendication 15, caractérisé en ce que le métabolite est l'IL$_2$.

EP 0 354 129 A1

1/5

FIG.1

Cellules x 10⁶/ml

1) ✳ MEM / F12+(I)+(T)
2) ☐ MEM / F12+ FIV1
4) △ MEM / F12+(I)+(T)  PVP3% PLURIOL 0,5‰
3) ◇ MEM / F12+(I)+(T)  PVP3%
5) ○ MEM / F12+(I)+(T)  PVP6% PLURIOL 0,5‰

jours

FIG.2

1) —✕— MEM / F 12+(I)+(T)
2) —▢— MEM / F 12+FIV 1
4) —△— MEM / F 12+(I)+(T)  PVP 3% PLURIOL 0,5‰
5) —◯— MEM / F 12+(I)+(T)  PVP 6% PLURIOL 0,5‰
3) —◇— MEM / F 12+(I)+(T)  PVP 3%

EP 0 354 129 A1

2/5

# FIG.3

Plasmide 832 (9230pb)

4/5

Productivité spécifique en hGH des cellules CHO

FIG. 4

☐ 4 jours

▨ 7 jours

Productivité spécifique en HBS des cellules CHO

FIG. 5

☐ 4 jours

▨ 7 jours

5/5

FIG.6

Productivité spécifique en hGH des cellules véro

μUI/uDO.J

□ 4 jours
▨ 7 jours

IT
1)
IT+PVP
2)
IT+PVP+PLURIOL
3)
FIVI
4)

FIG.7

Productivité spécifique en IgG d'hybridomes

ng/uDO.J

□ 4 jours
▨ 7 jours

IT
1)
IT+PVP
2)
IT+PVP+PLURIOL
3)

Office européen
des brevets

**RAPPORT DE RECHERCHE EUROPEENNE**

Numero de la demande

EP  89 40 2216

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | WO-A-8 800 967  (THE UNIVERSITY OF NEW SOUTH WALES)<br>* Revendications 1,2; page 3, lignes 31-35 *<br>--- | 1-1 | C 12 N    1/38<br>C 12 N    5/00 |
| A | CHEMICAL ABSTRACTS, vol. 110, 1989, page 546, abstract no. 55998b, Columbus, Ohio, US; & JP-A-63 84 487 (AGENCY OF INDUSTRIAL SCIENCES AND TECHNOLOGY) 15-04-1988<br>* Résumé *<br>--- | 1-1 | |
| A | US-A-3 827 942  (A.M. JANIK)<br>* Colonne 2, lignes 27-35 *<br>--- | 1-1 | |
| A | EP-A-0 239 292  (CELLTECH LTD)<br>* En entier *<br>----- | 1-1 | |

**DOMAINES TECHNIQUES RECHERCHES (Int. Cl.5)**

C 12 N

Le présent rapport a été établi pour toutes les revendications

| Lieu de la recherche | Date d'achèvement de la recherche | Examinateur |
|---|---|---|
| LA HAYE | 09-11-1989 | CUPIDO M. |